Europäisches Patentamt
European Patent Office
Office européen des brevets

(19)

(11) Publication number: **0 137 746**
**A2**

(12) EUROPEAN PATENT APPLICATION

(21) Application number: 84306256.3

(22) Date of filing: 13.09.84

(51) Int. Cl.[4]: C 07 C 109/02
C 07 C 109/04, C 07 C 149/23
C 07 C 149/273, C 07 C 153/07
C 07 D 295/22, C 07 K 5/06
A 61 K 31/195, A 61 K 31/265
A 61 K 31/395, A 61 K 37/02

(30) Priority: 22.09.83 GB 8325465
26.11.83 GB 8331643
07.02.84 GB 843217
07.02.84 GB 843218
12.05.84 GB 8412183

(43) Date of publication of application:
17.04.85 Bulletin 85/16

(84) Designated Contracting States:
AT BE CH DE FR GB IT LI LU NL SE

(71) Applicant: FISONS plc
Fison House Princes Street
Ipswich Suffolk IP1 1QH(GB)

(72) Inventor: Robinson, David Hulme
11 Countrymans Way
Shepshed Leicestershire(GB)

(74) Representative: Craig, Christopher Bradberry et al,
Fisons plc 12 Derby Road
Loughborough Leicestershire LE11 0BB(GB)

# (54) Nitrogen substituted angiotensin converting enzyme inhibitors.

(57) There are described compounds of formula I,

$$ZCHRCON\ (R_4NR_5)\ CHR_6COOH \qquad I$$

in which

Z is $R_2CH$ (COOH) NH– or $R_1SCH_2$–,

$R_1$ is hydrogen or $R_8CO$–,

$R_8$ is alkyl C1 to 10 or phenyl,

R is hydrogen, alkyl C1 to 6, or alkyl C1 to 6 substituted by phenyl, $-NH_2$, –COOH or by alkyl C1 to 6 thio,

$R_2$ is hydrogen, alkyl C1 to 10, cycloalkyl C3 to 10, phenyl, phenylalkyl C7 to 12, alkoxy C1 to 6 – alkyl C1 to 6, alkyl C1 to 6 – thioalkyl C1 to 6, phenyloxyalkyl C7 to 12 or phenylthioalkyl C7 to 12,

$R_4$ and $R_5$, which may be the same or different, are each hydrogen, alkyl C1 to 10, cycloalkyl C3 to 10, phenylalkyl C7 to 12, or phenyl; the phenyl and phenylalkyl each optionally being substituted by one or more alkyl C1 to 6, alkoxy C1 to 6 or halogen groups, or

$R_4$ and $R_5$ together form a $-(CH_2)_4-$ or $-(CH_2)_5-$ chain, and

$R_6$ is hydrogen, alkyl C1 to 6 or phenylalkyl C7 to 12, and pharmaceutically acceptable salts and esters thereof.

There are also described processes for making the compounds and their formulation and use as pharmaceuticals, eg. angiotensin converting enzyme inhibitors.

EP 0 137 746 A2

Croydon Printing Company Ltd

NITROGEN SUBSTITUTED ANGIOTENSIN CONVERTING ENZYME INHIBITORS

This invention relates to new compounds, methods for their preparation and compositions containing them.

A wide variety of angiotensin converting enzyme (ACE) inhibitors are known, e.g. from French Patent Specification No. 2,372,804 and European Patent Specification No. 0012401.

We have now found a group of compounds having advantageous properties, e.g. as ACE inhibitors.

According to the invention we provide compounds of formula I,

$$ZCHRCON(R_4NR_5)CHR_6COOH \qquad I$$

in which Z is $R_2CH(COOH)NH-$ or $R_1SCH_2-$,

$R_1$ is hydrogen or $R_8CO-$,

$R_8$ is alkyl C1 to 10 or phenyl,

R is hydrogen, alkyl C1 to 6, or alkyl C1 to 6 substituted by phenyl, $-NH_2$, -COOH or by alkyl C1 to 6-thio,

$R_2$ is hydrogen, alkyl C1 to 10, cycloalkyl C3 to 10, phenyl, phenylalkyl C7 to 12, alkoxy C1 to 6 - alkyl C1 to 6, alkyl C1 to 6 - thioalkyl C1 to 6, phenyloxyalkyl C7 to 12 or phenylthioalkyl C7 to 12,

$R_4$ and $R_5$, which may be the same or different,

are each hydrogen, alkyl C1 to 10, cycloalkyl C3 to 10, phenylalkyl C7 to 12, or phenyl; the phenyl and phenylalkyl each optionally being substituted by one or more alkyl C1 to 6, alkoxy C1 to 6 or halogen groups,

or $R_4$ and $R_5$ together form a $-(CH_2)_4-$ or $-(CH_2)_5-$ chain, and

$R_6$ is hydrogen, alkyl C1 to 6 or phenylalkyl C7 to 12,

and pharmaceutically acceptable salts, esters and amides thereof.

According to the invention we also provide a process for the production of a compound of formula I, or a pharmaceutically acceptable salt, ester or amide thereof, which comprises

a) removal of a protecting group from a compound of formula I in which one or more of the amino or carboxylic acid groups is protected,

b) production of a compound of formula I in which Z is $R_2CH(COOH)NH-$, or a salt or ester thereof, by reaction of a compound of formula II,

$$R_2CH(NH_2)COOR_3 \qquad II$$

in which $R_2$ is as defined above and $R_3$ is hydrogen or a protecting group,

with a compound of formula III,

$XCHRCON(R_4NR_5)CHR_6COOH$ III

or a salt, ester or protected derivative thereof,

in which R, $R_4$, $R_5$ and $R_6$ are as defined above, and

X is a good leaving group,

c) production of a compound of formula I in which $R_1$ is $R_8CO$- by reaction of a compound of formula IV,

$R_8CO\text{-}S\text{-}CH_2CHRCOX$ IV

in which X, R and $R_8$ are defined above,

with a compound of formula V,

$HN(NR_4R_5)CHR_6COOH$ V

or a salt, ester or protected derivative thereof,

in which $R_4$,$R_5$ and $R_6$ are as defined above, or

d) production of a compound of formula I in which $R_1$ is hydrogen by selective cleavage of a corresponding compound of formula I in which $R_1$ is $R_8CO$-,

and where desired or necessary deprotecting the resulting compound, or converting a compound of formula I to a pharmaceutically acceptable salt, ester or amide thereof or vice versa.

In process a) the protecting group can be any convenient protecting group conventionally used in peptide synthesis and may be removed using techniques conventionally used in peptide synthesis. Thus carboxy protecting groups which may be used are alkoxy, e.g. C 1 to 6 alkoxy such as t-butyloxy, or benzyloxy groups which can be removed, for example by hydrolysis, e.g. basic hydrolysis using aqueous methanolic sodium hydroxide; or cleavage using, for example, trifluoroacetic acid; or by hydrogenation. Amino-protecting groups which may be mentioned include benzyloxycarbonyl or t-butyloxycarbonyl. We prefer to use starting materials in which the carboxy groups are protected.

In process b) the group X may be, for example, halo, eg bromo or chloro. The reaction may be carried out in a solvent which is inert under the reaction conditions, e.g. acetonitrile, at a temperature of from 20° to 100°, preferably at about 80°C.

Process c) may be carried out at a temperature of from 0° to 50°C. The reaction may conveniently be carried out in a non-nucleophilic solvent under basic

conditions, eg in toluene with polyvinylpyridine.

In process d) the reaction is conveniently carried out in a solvent which is inert under the reaction conditions, e.g. methanol. The reaction is preferably carried out under basic conditions, e.g. in the presence of ammonia or potassium hydroxide.

The starting materials for the above processes are either known or may be made from known compounds using conventional processes. Thus compounds of formulae VI and VII,

$R_4R_5N\text{-}NHCHR_6COOR_3$ VI

RCHXCOX VII

may be reacted to yield a compound of formula III, eg under basic conditions for example polyvinylpyridine in toluene.

The compounds of formulae V and VI may be made by reacting a compound of formula VIII or IX.

$R_4R_5N\text{-}NH_2$ VIII

$R_4R_5N\text{-}NHCHO$ XI

with a compound of formula IX,

$XCHR_6COOR_3$ IX

and, when a compound of formula XI is used, removing the -CHO group, eg in refluxing ethanolic HCl.

In the above reaction scheme R, $R_3$, $R_4$, $R_5$, $R_6$ and X have the significances given earlier and the reactions may be carried out using conventional reaction conditions.

The compounds of formula I, and the intermediates therefor, may be isolated from their reaction mixtures using conventional techniques known per se.

The processes described above may produce the compound of formula I or a derivative thereof. It is also within the scope of this invention to treat any derivative so produced to liberate the free compound of formula I, or to convert one derivative into another.

In addition to the processes described above the compounds of formula I may be made by a variety of processes which are analogous to those known for the production of structurally similar compounds.

Pharmaceutically acceptable salts of the compounds of formula I include ammonium salts, alkali metal salts, e.g. sodium and potassium salts (which are preferred); alkaline earth metal salts, e.g. the calcium and magnesium

salts; salts with organic bases, e.g. salts with dicyclohexylamine or N-methyl-D-glucamine; and salts with amino acids, e.g. with arginine, lysine etc. Also, when Z is $R_2CH(COOH)NH-$, salts with organic or inorganic acids may be prepared, e.g. with HCl, HBr, $H_2SO_4$, $H_3PO_4$, methanesulfonic, toluensulfonic, maleic, fumaric or camphorsulfonic acids. The non-toxic physiologically acceptable salts are preferred, although other salts are also useful, e.g. in isolating or purifying the product. Pharmaceutically acceptable esters include alkyl C 1 to 6 esters and esters with benzyl alcohol. We specifically provide compounds in which Z is $R_2CH(COOH)NH-$ and the two -COOH groups are in different forms, e.g. where one is esterified and the other is not. More specifically we provide such compounds in which the -COOH group adjacent to the $R_2$ group is esterified.

The salts may be formed by conventional means, e.g. by reacting the free acid or free base forms of the product with one or more equivalents of the appropriate base or acid in a solvent or medium in which the salt is insoluble, or in a solvent such as water which is then removed in vacuo, or by freeze-drying, or by exchanging the ions of an existing salt for another ion on a suitable ion exchange resin. The esters may be made by using the appropriate starting materials in the reaction sequence

given above or by esterification of the free acid.

We prefer those compounds of formula I in which Z is $R_1SCH_2$-.

When Z is $R_2CH(COOH)NH$- we prefer the partial structure -NHCHRCO- in formula I to be part of a naturally occurring amino acid. Where any of R, $R_2$, $R_4$, $R_5$ or $R_8$ represent alkyl they may individually be straight, branched or cycloalkyl containing up to and including 6 carbon atoms, e.g. cyclohexyl. We prefer R to be hydrogen, benzyl, aminobutyl, carboxymethyl, methylthioethyl or alkyl C1 to 6, eg methyl. We also prefer $R_2$ to be alkyl C1 to 6, e.g. 3-methylbutyl, cycloalkyl C3 to 6, e.g. cyclohexyl; ethoxyalkyl; phenoxyethyl; phenylthioethyl; phenylalkyl C7 to 10, e.g. benzyl or phenylethyl or alkyl C1 to 4-thioalkyl C1 to 4, eg 2-methylthioethyl; and $R_4$ and $R_5$ to be alkyl C1 to 6, eg methyl or ethyl; phenylalkyl C7 to 10, eg benzyl; phenyl; halophenyl, eg 4-chlorophenyl or 3,4-dichlorophenyl; alkyl C1 to 6-phenyl, eg 4-methylphenyl; alkoxy C1 to 6 phenyl, e.g. 4-methoxyphenyl or together to form a $-(CH_2)_4-$ or $-(CH_2)_5-$ chain. We prefer $R_6$ to be hydrogen, methyl or phenylethyl. $R_1$ is preferably hydrogen, benzoyl or alkanoyl C2 to 6, eg acetyl or pivaloyl. A specific group of compounds of formula I are those in which $R_2$ is

cycloalkyl C3 to 10 or phenythioalkyl C7 to 12.

In those compounds of formula I in which Z is $R_1SCH_2$ - we prefer the carboxy group to be in the form of the free acid, or a pharmaceutically acceptable salt thereof.

As a specific group of compounds of formula I we provide compounds of formula Ia,

$R_2CH(COOH)NHCHRCON(NR_4R_5)CHR_6COOH$ Ia

in which R is hydrogen or alkyl C1 to 6,

$R_2$ is hydrogen, alkyl C1 to 10 or phenylalkyl C7 to 12,

$R_4$ and $R_5$, which may be the same or different, are each hydrogen, alkyl C1 to 10, cycloalkyl C3 to C10, phenylalkyl C7 to 12, or phenyl, the phenyl and phenylalkyl each optionally being substituted by one or more alkyl C1 to 6, alkoxy C1 to 6 or halogen groups,

or $R_4$ and $R_5$ together form a $-(CH_2)_4-$ or $-(CH_2)_5-$ chain, and

$R_6$ is hydrogen or alkyl C1 to 6,

and pharmaceutically acceptable salts and esters thereof.

As a further specific group of compounds of formula I we provide compounds of formula Ib,

$R_1SCH_2CHR-CON(NR_4R_5)CHR_6COOH$ Ib

in which $R_1$ is hydrogen or $R_8CO-$,

$R_8$ is alkyl C1 to 10, or phenyl,

R is hydrogen or alkyl C1 to 6,

$R_4$ and $R_5$, which may be the same or different, are each, hydrogen, alkyl C1 to 10, cycloalkyl C3 to 10, phenylalkyl C7 to 12, or phenyl; the phenyl and phenylalkyl each optionally being substituted by one or more alkyl C1 to 6, alkoxy C1 to 6 or halogen groups,

or $R_4$ and $R_5$ may together form a $-(CH_2)_4-$ or $-(CH_2)_5-$ chain, and

$R_6$ is hydrogen, or alkyl C1 to 10,

and pharmaceutically acceptable salts and esters thereof.

As a still further specific group we provide compounds of formula Ic,

$R_1SCH_2CHR-CON(NR_4R_5)CH R_6COOH$ Ic

in which $R_1$ is hydrogen or $R_8CO-$,

$R_8$ is alkyl C1 to 10, or phenyl,

R is hydrogen or alkyl C1 to 10,

$R_4$ and $R_5$, which may be the same or different, are each, alkyl C1 to 10, phenylalkyl C7 to 12, or phenyl; the phenyl optionally being substituted by one or more

alkyl C1 to 6, alkoxy C1 to 6 or halogen groups, and

$R_6$ is hydrogen, or alkyl C1 to 10,

and pharmaceutically acceptable salts, esters and amides thereof.

As a yet further specific group we provide compounds of formula Id,

$R_2CH(COOH)NHCHRCON(NR_4R_5)CH_2COOH$ Id

in which R is hydrogen, alkyl C1 to 6, or alkyl C1 to 6 substituted by phenyl, $-NH_2$, -COOH or by alkyl C1 to 6-thio,

$R_2$ is hydrogen, alkyl C1 to 10, cycloalkyl C3 to 10, phenyl, phenylalkyl C7 to 12, alkoxy C1 to 6 - alkyl C1 to 6, alkyl C1 to 6 - thioalkyl C1 to 6, phenyloxyalkyl C7 to 12 or phenylthioalkyl C7 to 12,

$R_4$ and $R_5$, which may be the same or different, are each hydrogen, alkyl C1 to 6, phenylalkyl C7 to 12, or phenyl, the phenyl optionally being substituted by one or more alkyl C1 to 6, alkoxy C1 to 6 or halogen groups, or $R_4$ and $R_5$ together form a $-(CH_2)_4-$ chain,

and pharmaceutically acceptable salts, esters and amides thereof.

The compounds of formula I may contain one or more asymmetric carbon atoms and may therefore exhibit optical and/or diastereo isomerism. We have also, surprisingly, found that certain of the compounds form stable isomers

(rotamers) resulting from restricted rotation about the N-N bond. Diastereoisomers and rotamers may be separated using conventional techniques, e.g. chromatography or fractional crystallisation. The various optical isomers may be made by separation of a racemic or other mixture of the compounds using conventional, eg. fractional crystallisation or HPLC, techniques. Alternatively the desired optical isomers may be made by reaction of the appropriate optically active starting materials under conditions which will not cause racemisation. We prefer those compounds of formula I in which any asymmetric carbon atoms are in the S configuration.

The compounds of the invention are advantageous in that they are more efficaceous, produce less side effects, are longer acting, more readily absorbed, less toxic, distributed in the body tissues in a different manner or have other advantageous properties when compared to compounds of similar structure.

The compounds of the invention are useful because they possess pharmacological properties. In particular they inhibit angiotensin converting enzyme and thus block conversion of the decapeptide angiotensin I to angiotensin II (see Example A). Angiotensin II is a potent vasoconstrictor in mammals. It also stimulates aldosterone release which results in salt and fluid

retention. Increased blood pressure is the physiological result of these changes. Inhibitors of angiotensin converting enzyme are thus effective antihypertensive agents in a variety of animal models (see Example B) and are indicated for use clinically, for example, in patients with renovascular, malignant or essential hypertension or chronic congestive heart failure. See, for example, D W Cushman et al., Biochemistry 16, 5484 (1977) and E W Petrillo and M A Ondetti, Med. Res. Rev. 2 93 (1982).

Thus, the compounds of this invention are useful as antihypertensives in treating hypertensive mammals, including humans and they can be utilised to achieve redution of blood pressure, e.g. in formulations containing appropriate pharmaceutically acceptable excipients, diluents or carriers. The compounds of the invention can be administered (to animals or humans) in unit dosages of 5 to 500mg generally given several times, e.g. 1 to 4 times, per day thus giving a total daily dose of from 5 to 2000 mg per day. The dose will vary depending on the type and severity of disease, weight of patient and other factors which a person skilled in the art will recognise.

The compounds of this invention may be given in combination with other pharmaceutically active compounds, e.g. diuretics or antihypertensives. The dosage of the

other pharmaceutically active compound can be that conventionally used when the compound is administered on its own, but is preferably somewhat lower. To illustrate these combinations, one of the antihypertensives of this invention effective clinically in the range, e.g. 15-200 milligrams per day, can be combined at levels ranging, e.g. from 3-200 milligrams per day with the following antihypertensives and diuretics in dose ranges per day as indicated:

hydrochlorothiazide (15-200mg), chlorothiazide (125-2000mg), ethacrynic acid (15-200mg), amiloride (5-20mg), furosemide (5-80mg), propanolol (20-480mg), timolol (5-50mg) nifedipine (20-100mg), verapamil (120-480mg) and methyldopa (65-2000mg). In addition, the triple drug combinations of hydrochlorothiazide (15-200mg) plus amiloride (5-20mg) plus converting enzyme inhibitor of this invention (3-200mg) or hydrochlorothiazide (15-200mg) plus timolol (5-50mg), plus the converting enzyme inhibitor of this invention (3-200mg) are contemplated. The above dose ranges may be adjusted on a unit basis as necessary to permit divided daily dosage. Also, the dose may vary depending on the severity of the disease, weight of patient and other factors which a person skilled in the art will recognise.

According to our invention we also provide a

pharmaceutical composition comprising preferably less than 80%, more preferably less than 50%, eg 1 to 20%, by weight of a compound of formula I, or a pharmaceutically acceptable salt or ester thereof, in admixture with a pharmaceutically acceptable adjuvant, diluent or carrier.

Thus the compound may be put up as a tablet, capsule, dragee, suppository, suspension, solution, injection, implant, a topical, eg transdermal, preparation such as a gel, cream, ointment, aerosol or a polymer system, or an inhalation form, e.g. an aerosol or a powder formulation.

We prefer compositions which are designed to be taken oesophageally and to release their contents in the gastrointestinal tract. Thus we prefer tablets which may, for example, be made by direct compression. In such a process the active ingredient is mixed with one or more of modified forms of starch, calcium phosphate, a sugar eg lactose, microcrystalline cellulose and/or other directly compressible excipients, together with lubricant(s), eg stearic acid or magnesium stearate, flow aid(s), eg talc or colloidal silicon dioxide, and disintegrant(s), eg starch or the materials sold under the Trade Marks, Nymcel, Ac-Di-Sol, Explotab and Plasdone XL. Tablets are then formed by direct compression, and may be sugar or film coated e.g. with hydroxypropylmethylcellulose.

Alternatively the active ingredient may be granulated

before tabletting. In such cases the active ingredient is mixed with one or more of starch, calcium phosphate, a sugar eg lactose, microcrystalline cellulose or other suitable excipients and granulated with a binder such as starch, pregelled starch, polyvinylpyrrolidone, gelatine, a modified gelatine, or a cellulose derivative, eg hydroxypropylmethylcellulose. The mass is then dried, sieved and mixed with lubricant(s), flow aid(s) and disintegrant(s), such as described in the previous paragraph. Tablets are then formed by compression of the granules, and may be sugar or film coated, eg with hydroxypropylmethylcellulose.

As a further alternative a powder, blend or granules, such as are described above as intermediates in tabletting, may be filled into a suitable, eg gelatine, capsule.

In order to improve the bioavailability, or decrease variability of availability, of the active ingredient the compound may be:-

a) dissolved in a suitable solvent, eg polyethylene glycol, Gelucaire, arachis oil, a (hydrogenated) vegetable oil or beeswax and the solution is then filled into a gelatine capsule,

b) produced as a spray-dried or freeze-dried form prior to mixing with other excipients,

c) milled and/or micronised to produce a powder with a large surface area prior to mixing with other excipients,

d) made into a solution and distributed over an inert excipient having a large surface area, eg colloidal silicon dioxide. The solvent is evaporated and further excipients added,

e) formed into a complex with cyclodextrin prior to mixing with other excipients. This complex also assists in increasing light stability, or

f) made into a solid solution or co-precipitated, eg with polyvinylpyrrolidone, polyethyleneglycol, modified cellulose, hydroxypropylmethylcellulose, urea or a sugar prior to mixing with further excipients.

The compounds, either in their normal form or in a modified form, eg as described immediately above, may be formulated in a controlled release form. Thus the compound may be dispersed, or contained in, a polymer matrix formed from, for example, ethylcellulose, hydroxypropylmethylcellulose or the product sold under the Trade Mark Eudragit. Alternatively the compound may be formulated as a tablet or beads which are surrounded by a semi-permeable membrane, eg shellac, ethylcellulose or an acrylate/methacrylate polymer.

Certain of the compounds of formula I can form hydrates or solvates, e.g. with an alcohol such as ethanol.

The invention is illustrated, but in no way limited by the following Examples in which temperatures are in degrees centigrade.

Example 1

(S)[1-[N-(1-Carboxy-2-phenylethyl)glycyl]-2-methyl-2-phenylhydrazino]acetic acid

a) Benzyl (2-methyl-2-phenylhydrazino)acetate

A solution of 1-methyl-1-phenylhydrazine (36.4g) and benzyl bromoacetate (23.8g) in dry methanol (200ml) was stirred under nitrogen at room temperature for 48 hours. The mixture was evaporated to an oil. A solution of the residue in ether (200ml) was washed with dilute hydrochloric acid and water. The dried ($Na_2SO_4$) organic phase was evaporated to dryness and the residue purified by chromatography on silica gel. Trituration of the resulting oil with petroleum ether (bp 40-60$^o$) gave the sub-title product (10.0g) as cream coloured crystals, mp 34-36$^o$.

b) Benzyl [1-(2-chloro-1-oxoethyl)-2-methyl-2-phenylhydrazino]acetate

A solution of chloroacetyl chloride (0.64ml) in toluene (20ml) was added to a mixture of the product of step a) (1.08g) and polyvinylpyridine (3.2g) in toluene and the mixture stirred at room temperature under nitrogen for 2 hours. Water (25ml) was added and the mixture

stirred for a further 1 hour. The solid was removed by filtration and washed with ether. The combined organic solutions were washed with sodium bicarbonate solution, water, dried ($Na_2SO_4$) and evaporated to give the sub-title product as a colourless oil (1.4g).

c) Benzyl (S)-[1-N-(1-benzyloxycarbonyl-2-phenylethyl)-glycyl]-2-methyl-2-phenylhydrazino]acetate

A solution of the product of step (b) (1.5g) and L-phenylalanine benzyl ester (2.21g) in acetonitrile was heated at reflux for 48 hours. The mixture was filtered and the filtrate evaporated to dryness. A solution of the residue in ether was washed with water, dried ($Na_2SO_4$) and evaporated to a pale yellow oil (3.5g). The oil was purified by silica gel chromatography to give the sub-title product (1.6g) as a colourless gum.

$C_{34}H_{35}N_3O_5$

Requires C72.19, H6.24, N7.43%

Found C72.46, H6.24, N7.03%

Molecular weight 565

Mass spectrum shows m/z 566 ($M^+$+1)

d) (S)[1-[N-(1-Carboxy-2-phenylethyl)glycyl]-2-methyl-2-phenylhydrazino]acetic acid

A solution of the product of step c) (1.4g) in ethanol was hydrogenated at atmospheric pressure for 5 hours using 10% paladium/carbon (0.2g) as catalyst. The

catalyst was removed by filtration and the filtrate evaporated to dryness. A solution of the residue in ether was washed with water, dried ($Na_2SO_4$) and evaporated to give the title compound (0.9g) as white crystals, mp 115° (dec). The product contained ethanol of crystallisation.

$C_{20}H_{23}N_3O_5$ $C_2H_6O$

Requires C61.25, H6.73, N9.75%

Found C61.14, H6.15, N10.12%

Molecular weight 385

Mass spectrum (fast atom bombardment) shows m/z 386 ($M^+$+1).

Example 2

(S)-[1-[N-[1-Carboxy-3-(methylthio)propyl]-glycyl]-2-methyl-2-phenylhydrazino]acetic acid

a) Benzyl (S)[1-[N-[1-methoxycarbonyl-3-(methylthio) propyl]glycyl]-2-methyl-2-phenylhydrazino]acetate

Prepared using appropriate starting materials and the process of Example 1c). Product isolated as a colourless oil.

$C_{24}H_{31}N_3O_5S$

Requires C60.90, H6.55, N8.88, S6.77%

Found C60.95, H6.71, N8.81, S6.78%

Molecular weight 473

Mass spectrum (fast atom bombardment) shows m/z 474

($M^+$+1)

b) Disodium (S)[1-[N-[1-carboxy-3-(methylthio)propyl] glycyl]-2-methyl-2-phenylhydrazino]acetate

A solution of the product of step a) (355mg) in a mixture of methanol (10ml) and water (4ml) was treated with 1N sodium hydroxide solution (1.5ml). After 24h at room temperature the solvent was removed *in vacuo*. Trituration of the residue with ether yielded the title compound (250mg) as a white powder, m.p.> 300°. The product was a dihydrate.

$C_{16}H_{21}N_3O_5S\ Na_2.\ 2H_2O$.

Requires C42.76, H5.56, N9.35, S7.13%

Found C42.42, H4.96, N8.74, S7.13%

Molecular weight 413.

Mass spectrum (fast atom bombardment) shows m/z 414.

Example 3

[1-[N-(1-Carboxy-3-phenylpropyl)glycyl]-2-methyl-2-phenylhydrazino]acetic acid

a) Benzyl[1-[N-(1-benzyloxycarbonyl-3-phenylpropyl)-glycyl]-2-methyl-2-phenylhydrazino]acetate

Prepared using appropriate starting materials and the process of Example 1c).

Product isolated as a colourless oil.

$C_{35}H_{37}N_3O_5$

Requires C71.96, H6.53, N7.41%

Found C72.23, H6.44, N7.20%

Molecular weight 579.

Mass spectrum shows m/z 580 ($M^+$+1).

b) [1-(N-(1-Carboxy-3-phenylpropyl)glycyl)-2-methyl-2-phenylhydrazino]acetic acid

Prepared using the product of step a) and the process of Example 1d), but using 95% aqueous acetic acid as solvent. The product was isolated as a dihydrate.

$C_{21}H_{25}N_3O_5.2H_2O$

Requires C57.93, H6.20, N9.65%

Found C58.03, H5.88, N9.38%

Molecular weight 399.

Mass spectrum (fast atom bombardment) shows m/z 400 ($M^+$+1)

Mass spectrum (electron impact) shows m/z 381 ($M^+$-18).

Example 4

Benzyl [1-[N-(1-benzyloxycarbonyl-2-phenylethyl)-alanyl]-2-methyl-2-phenylhydrazino]acetate

a) Benzyl [1-(2-bromo-1-oxopropyl)-2-methyl-2-phenylhydrazino]acetate

Prepared according to the process of Example 1b) using 2-bromopropionyl chloride. The product was isolated as a colourless oil.

Molecular weight 405.

Mass spectrum shows m/z 404/406.

• b) <u>Benzyl [1-(N-(1-benzyloxycarbonyl-2-phenylethyl)-alanyl)-2-methyl-2-phenylhydrazino]acetate</u>

A solution of the product of step a) (300mg) and L-phenylalanine benzyl ester (380mg) in acetonitrile (5ml) was heated at reflux for 3 days. The mixture was filtered and a solution of the filtrate in ether was washed with water, dried ($Na_2SO_4$) and evaporated to a yellow oil. Analytical HPLC showed the presence of 4 major components. These were separated by preparative HPLC on silica using ethyl acetate and hexane as solvents to yield 4 separate product isomers.

<u>Isomer 1</u>

A colourless gum

$C_{35}H_3N_3O_5$ - Molecular weight 579.

Mass spectrum (fast atom bombardment) shows m/z 580 ($M^+$+1).

The n.m.r. spectrum (360$MH_Z$) in $CDCl_3$ shows a characteristic doublet at $\delta$ 5.35 (1H).

<u>Isomer 2</u>

A colourless gum

Mass spectrum (fast atom bombardment) shows m/z 580 ($M^+$+1).

The n.m.r. spectrum (360$MH_Z$) in $CDCl_3$ shows a characteristic doublet at $\delta$ 5.29 (1H).

<u>Isomer 3</u>

A colourless gum

Mass spectrum (fast atom bombardment) shows m/z 580 ($M^+$+1).

The n.m.r. spectrum ($360MH_z$) in $CDCl_3$ shows a characteristic doublet at $\delta$ 5.37 (1H).

Isomer 4

A colourless gum

Mass spectrum (fast atom bombardment) shows m/z 580 ($M^+$+1).

The n.m.r. spectrum ($360MH_z$) in $CDCl_3$ shows a characteristic doublet at $\delta$ 5.32 (1H).

Example 5

Benzyl [1-(N-(1-benzyloxycarbonyl-3-phenylpropyl) alanyl)-2-methyl-2-phenyl-hydrazino]acetate

Prepared according to the process of Example 4b) using appropriate starting materials. The mixture of 4-isomers identified by (HPLC) was isolated as a colourless oil.

$C_{36}H_{39}N_3O_5$ Molecular weight 593.

Mass spectrum (fast atom bombardment) shows m/z 594 ($M^+$+1).

Example 6

[1-(3-Mercapto-1-oxopropyl)-2-methyl-2-phenylhydrazino]-acetic acid

a) Benzyl [1-(3-benzoylthio-1-oxopropyl)-2-methyl-2-phenylhydrazino]acetate

A solution of the product of Example 1a) (1.08g) and 3-(benzoylthio)propanoyl chloride (1.84g) in dry toluene (40ml) was treated with polyvinylpyridine (3.2g) and the mixture stirred under nitrogen for 2h. The solid was removed by filtration and the filtrate stirred vigorously with water (10ml) for 3h. The organic phase was washed with saturated sodium bicarbonate solution and water, dried and evaporated to an oil. The residue was purified by chromatography on silica gel to give the sub-title product (1.6g) as a colourless gum.

$C_{26}H_{26}N_2O_4S$. Molecular weight 462.

Mass spectrum shows m/z 462 ($M^+$).

b) [1-(3-Mercapto-1-oxopropyl)-2-methyl-2-phenylhydrazino] acetic acid dicyclohexylamine salt

A solution of potassium hydroxide (0.215g) in methanol (5ml) was added dropwise to a solution of the product of step a) (1.5g) in methanol (25ml) at $0^o$ under nitrogen and the mixture stirred for 30 min. Ether (10ml), pentane (10ml) and water (15ml) were added to the mixture and the organic phase separated and discarded. A solution of potassium hydroxide (0.263g) in water (2ml) was added to the aqueous layer and the mixture stirred at room temperature under nitrogen for 2h. The solution was washed with ethyl acetate, acidified with dilute

hydrochloric acid and extracted with ethyl acetate. The extract was dried ($Na_2SO_4$) and evaporated to a colourless oil (0.9g). A solution of the oil in ether was treated with dicyclohexylamine (0.65ml) and the mixture evaporated to a pale yellow solid. Recrystallisation of the residue from ethanol yielded the title compound (0.5g) as white crystals, mp 167-170$^{o}$.

$C_{12}H_{16}N_2O_3S$. $C_{12}H_{23}N$

Requires C64.14, H8.69, N9.35, S7.13%

Found C64.27, H8.48, N9.18, S6.80%

Molecular weight of acid 268.

Mass spectrum showed m/z 268 ($M^+$).

Example 7

[1-(3-Mercapto-2-methyl-1-oxopropyl)-2-methyl-2-phenyl-hydrazino]acetic acid

a) Benzyl [1-(3-benzoylthio-2-methyl-1-oxopropyl)-2-methyl-2-phenylhydrazino]acetate

Prepared using appropriate starting materials and the process of Example 6a). The product was isolated as a clear oil.

$C_{27}H_{28}N_2O_4S$.

Requires C68.07, H5.88, N5.88, S6.72%

Found C68.22, H6.06, N5.77, S6.40%

Molecular weight 476.

Mass spectrum showed m/z 476 ($M^+$).

b) [1-(3-Mercapto-2-methyl-1-oxopropyl)-2-methyl-2-phenylhydrazino]acetic acid

A solution of potassium hydroxide (0.35g) in methanol was added dropwise to a solution of the product of step a) (2.5g) in methanol (40ml) at $0^{o}$ under nitrogen. After stirring for 0.5h at $5^{o}$ a mixture of ether (20ml), petroleum ether (20ml) and water (30ml) were added to the reaction mixture. The aqueous phase was removed and treated dropwise with a solution of potassium hydroxide (0.425g) in water (4ml). The reaction mixture was then stirred under nitrogen at room temperature for 2h. The resulting mixture was washed with ethyl acetate, acidified with 2M hydrochloric acid and the product extracted with ethyl acetate. The organic solution was dried ($Na_2SO_4$) and evaporated to an oil. Trituration of the residue with cyclohexane yielded the title compound (0.7g) as white crystals, mp 140-$3^{o}$.

$C_{13}H_{18}N_2O_3S$.

Requires C55.32, H6.38, N9.93, S11.35%

Found C55.53, H6.32, N9.71, S11.00%

Molecular weight of acid 282.

Mass spectrum showed m/z 282 ($M^+$).

Example 8

[1-(3-Benzoylthio-2-methyl-1-oxopropyl)-2-methyl-2-phenylhydrazino]acetic acid

a) tert-Butyl [2-methyl-2-phenylhyrazino]acetate

Prepared from N-methyl-N-phenylhydrazine and tert-butyl bromoacetate by the method of Example 1a). The product was isolated as cream coloured crystals, mp 48-50°.

b) tert-Butyl [1-(3-benzoylthio-2-methyl-1-oxopropyl)-2-methyl-2-phenylhydrazino]acetate

Prepared from the product of step a) according to the process of Example 6a). The product was isolated as a colourless oil.

$C_{24}H_{30}N_2O_4S$. Molecular weight 442.

Mass spectrum shows m/z 442 ($M^+$).

c) [1-(3-Benzoylthio-2-methyl-1-oxopropyl)-2-methyl-2-phenylhydrazino]acetic acid

A solution of the product of step b) (0.9g) in dichloromethane (10ml) was treated with trifluoroacetic acid (2.5ml) and the mixture stirred at room temperature for 16h. The solution was evaporated to give a cream coloured solid. Recrystallisation of the residue from ethyl acetate yielded the title compound (0.45g) as white crystals, mp 161-3°.

$C_{20}H_{22}N_2O_4S$

| | |
|---|---|
| Requires | C62.18, H5.70, N7.25% |
| Found | C62.35, H5.79, N7.15% |

Molecular weight 386

Mass spectrum shows m/z 386 ($M^+$).

Example 9

[2-(4-Chlorophenyl)-1-(3-mercapto-2-methyl-1-oxopropyl)-2-methylhydrazino]acetic acid

a) 2-(4-Chlorophenyl)-1-formyl-2-methylhydrazine

Acetic anhydride (60ml) was added dropwise to a solution of 1-(4-chlorophenyl)-1-methylhydrazine (7.4g) in formic acid (130ml) at $0^o$. The mixture was allowed to warm to room temperature over 0.5h, poured into ice water and stirred for 2h. The product was extracted into chloroform, the organic solution washed with sodium bicarbonate solution and water, dried ($Na_2SO_4$) and evaporated to give the sub-title compound (8.2g) as white crystals, mp 75-$7^o$.

Molecular weight 184.5

Mass spectrum shows m/z 184/6.

b) Ethyl [2-(4-chlorophenyl)-1-formyl-2-methylhydrazino] acetate

Ethyl bromoacetate (8.17g) was added dropwise to a stirred mixture of the product of step a) (8.2g) and potassium carbonate (6.7g) in dimethylformamide (50ml). The mixture was stirred for 1h, poured into water and the product extracted into chloroform. The extract was dried ($Na_2SO_4$) and evaporated to give the sub-title product (10.4g) as an oil.

$C_{12}H_{15}ClN_2O_3$

Molecular weight 270.5

Mass spectrum shows 270/2.

c) Ethyl [1-(3-benzoylthio-2-methyl-1-oxopropyl)-2-(4-chlorophenyl)-2-methylhydrazino]acetate

A solution of the product of step b) (10.4g) in 1M ethanolic HCl (120ml) was heated at reflux for 1.5h. The mixture was evaporated to give ethyl [2-(4-chlorophenyl)-2-methylhydrazino] acetate hydrochloride as an oil. A suspension of the oil (5.0g), polyvinylpyridine (8.0g) and 3-benzoylthio-2-methylpropanoyl chloride (4.75g) in toluene (250ml) was stirred for 18h. The mixture was filtered and the filtrate evaporated to an oil. Trituration of the residue with ether/petroleum ether gave the sub-title product (4.2g) as white crystals, mp 102-3°.

$C_{22}H_{25}Cl\ N_2O_4S$

Requires C58.86, H5.57, N6.24, S7.13, Cl 8.04%

Found C58.98, H5.59, N6.39, S7.21, Cl 7.91%

Molecular weight 448.5

Mass spectrum shows m/z 448/450

d) [2-(4-Chlorophenyl)-1-(3-mercapto-2-methyl-1-oxopropyl)-2-methylhydrazino]acetic acid

Prepared from the product of step c) using the process of Example 7b). Product isolated as colourless

crystals, mp 136-7°.

$C_{13}H_{17}ClN_2O_3S$

Requires C49.29, H5.37, N8.84, S10.11, Cl 11.21%

Found C49.36, H5.33, N8.73, S10.15, Cl 11.37%

Molecular weight 316.5

Mass spectrum shows m/z 316/8

Example 10

(S)-[1-(3-Acetylthio-2-methyl-1-oxopropyl)-2-methyl-2-phenylhydrazino]acetic acid

a) tert-Butyl (S)-[1-(3-acetylthio-2-methyl-1-oxopropyl)-2-methyl-2-phenylhydrazino]acetate

Prepared according to the process of Example 6a) using appropriate starting materials. Product isolated as an oil.

$C_{19}H_{28}N_2O_4S$

Requires C60.00, H7.37, N7.37, S8.42%

Found C60.02, H7.31, N7.23, S8.75%

Molecular weight 380.

Mass spectrum shows m/z 380 ($M^+$).

b) (S)-[1-(3-Acetylthio-2-methyl-1-oxopropyl)-2-methyl-2-phenylhydrazino]acetic acid

Prepared from the product of step a) using the process of Example 8c). Product was isolated as colourless crystals, mp 98-100°.

$C_{15}H_{20}N_2O_4S$

Requires C55.56, H6.17, N8.64, S 9.88%

Found C55.55, H6.14, N8.49, S10.20%

Molecular weight 324.

Mass spectrum shows m/z 324 ($M^+$).

Example 11

(S)-[1-(3-Mercapto-2-methyl-1-oxopropyl)-2-methyl-2-phenylhydrazino]acetic acid

a) Benzyl (S)-[1-(3-benzoylthio-2-methyl-1-oxopropyl)-2-methyl-2-phenylhydrazino]acetate

Prepared according to the process of Example 6a) using appropriate starting materials. The product was isolated as a clear oil.

$C_{27}H_{28}N_2O_4S$

Requires C68.07, H5.88, N5.88, S6.72%

Found C68.06, H6.13, N6.09, S6.34%

Molecular weight 476.

Mass spectrum shows m/z 476 ($M^+$).

b) (S)-[1-(3-Mercapto-2-methyl-1-oxopropyl)-2-methyl-2-phenylhydrazino]acetic acid

Prepared according to the process of Example 7b) using appropriate starting materials. The product was isolated as colourless crystals, mp 141-4°.

$C_{13}H_{18}N_2O_3S$

Requires C55.32, H6.38, N9.93, S11.35%

Found C55.25, H6.32, N9.79, S11.35%

Molecular weight 282.

Mass spectrum shows M/z 282.

Example 12

[1-(3-Mercapto-2-methyl-1-oxopropyl)-2-methyl-2-(4-methylphenyl)hydrazino]acetic acid

a) 1-Formyl-2-methyl-2-(4-methylphenyl)hydrazine

Prepared from appropriate starting materials by the process of Example 9a). The product was isolated as an oil.

$C_9H_{12}N_2O$ Molecular weight 164

Mass spectrum shows m/z 164 ($M^+$).

b) Ethyl [1-formyl-2-methyl-2-(4-methylphenyl) hydrazino]acetate

Prepared from the product of step a) by the process of Example 9b). The product was isolated as an oil.

$C_{13}H_{18}N_2O_3$ Molecular weight 250.

Mass spectrum shows m/z 250 ($M^+$).

c) Ethyl [1-(3-benzoylthio-2-methyl-1-oxopropyl)-2-methyl-2-(4-methylphenyl)-hydrazino]acetate

Prepared from the product of step b) by the process of Example 6a). The product was isolated as colourless crystals, m.p. 80°.

$C_{23}H_{28}N_2O_4S$.

| | |
|---|---|
| Requires | C64.48, H6.54, N6.54, S7.47% |
| Found | C64.08, H6.21, N6.35, S7.67% |

Molecular weight 428.

Mass spectrum shows m/z 428 ($M^+$).

d) [1-(3-Mercapto-2-methyl-1-oxopropyl)-2-methyl-2-(4-methylphenyl)hydrazino]acetic acid

Prepared from the product of step c) by the process of Example 7b). The product was isolated as colourless crystals, mp 109-110°.

$C_{14}H_{20}N_2O_3S$.

Requires C56.75, H6.75, N9.46, S10.81%

Found C56.80, H6.84, N9.24, S10.92%

Molecular weight 296.

Mass spectrum shows m/z 296 ($M^+$).

Example 13

2-[1-(3-Mercapto-1-oxopropyl)-2-methyl-2-phenylhydrazino]-propanoic acid

a) 1-Formyl-2-methyl-2-phenylhydrazine

Prepared from appropriate starting materials using the process of Example 9a). The product was isolated as a clear oil.

$C_8H_{10}N_2O$ Molecular weight 150.

Mass spectrum shows m/z 150 ($M^+$).

b) Ethyl 2-(1-formyl-2-methyl-2-phenylhydrazino) propanoate

Prepared from the product of step a) using the process of Example 9b). The product was isolated as an

oil.

c) Ethyl 2-[1-(3-benzoylthio-1-oxopropyl)-2-methyl-2-phenylhydrazino]propanoate

Prepared from the product of step b) using the process of Example 6a). The product was isolated as a clear oil.

$C_{22}H_{26}N_2O_4S$.

Requires C63.77, H6.28, N6.76%

Found C63.21, H6.34, N6.31%

Molecular weight 414.

Mass spectrum shows m/z 414 ($M^+$).

d) 2-[1-(3-Mercapto-1-oxopropyl)-2-methyl-2-phenylhydrazino]propanoic acid dicyclohexylamine salt

Prepared from the product of step c) using the process of Example 6b). The product was isolated as colourless crystals, mp 150-152°.

$C_{13}H_{18}N_2O_3S$. $C_{12}H_{23}N$

Requires C64.79, H8.86, N9.07, S6.91%

Found C64.65, H8.55, N8.97, S7.17%

Molecular weight 282.

Mass spectrum shows m/z 282 ($M^+$).

Example 14

2-[1-(3-Mercapto-1-oxopropyl)-2-methyl-2-phenylhydrazino]-4-phenylbutyric acid

a) Ethyl 2-[1-(3-benzoylthio-1-oxopropyl)-2-methyl-2-

• phenylhydrazino]-4-phenylbutyrate

Prepared from appropriate starting materials and the process of Example 6a). The product was isolated as an oil.

$C_{29}H_{32}N_2O_4S$ Molecular weight 504.

Mass spectrum shows m/z 504 ($M^+$).

b) 2-[1-(3-Mercapto-1-oxopropyl)-2-methyl-2-phenylhydrazino]-4-phenylbutyric acid

Prepared from the product of step a) using the process of Example 6b). The compound was obtained as a clear gum.

$C_{20}H_{24}N_2O_3S$ Molecular weight 372.

Mass spectrum shows m/z 372 ($M^+$).

Example 15

[1-(3-Mercapto-2-methyl-1-oxopropyl)-2,2-dimethylhydrazino] acetic acid

a) 1-Formyl-2,2-dimethylhydrazine

A solution of 1,1-dimethylhydrazine (20g) and methyl formate (26ml) in methanol (30ml) was heated at reflux for 6h. The mixture was evaporated to a semi-solid residue. Trituration of the material with cold ether gave the product (22.2g) as white crystals, mp 60-63°.

$C_3H_8N_2O$. Molecular weight 88.

Mass spectrum shows m/z 88 ($M^+$).

b) Ethyl [1-formyl-2,2-dimethyl-1-hydrazino]acetate

A solution of the product of step a) (13.6g) in dry dimethylformamide (100ml) was added dropwise to a suspension of sodium hydride (4.08g) in dry dimethylformamide at $20^{o}$. The mixture was stirred under nitrogen for 0.5h at room temperature. A solution of ethyl bromoacetate (28.39g) in dimethylformamide (50ml) was added dropwise, and the mixture stirred at room temperature for 16h. The solvent was removed by evaporation and the product partitioned between ethyl acetate and water. The aqueous phase was washed with ethyl acetate (3x). The combined organic solutions were dried ($Na_2SO_4$) and evaporated to an oil. The product was purified by chromatography on silica gel using dichloromethane:ethanol as eluent. The material was isolated as an oil (12.3g).

$C_7H_{14}N_2O_3$ Molecular weight 174.

Mass spectrum shows m/z 175 ($M^+$+1).

c) <u>Ethyl [1-(3-benzoylthio-2-methyl-1-oxopropyl)-2,2-dimethylhydrazino]acetate</u>

Prepared from the product of step b) by the process of Example 9c). The product was isolated as an oil.

$C_{17}H_{24}N_2O_4S$ Molecular weight 352.

| | |
|---|---|
| Requires | C57.95, H6.82, N7.95, S9.09% |
| Found | C58.21, H6.71, N8.20, S8.81% |

Mass spectrum shows m/z 353 ($M^+$+1).

d) [1-(3-Mercapto-2-methyl-1-oxopropyl)-2,2-dimethylhydrazino]acetic acid

A solution of the product of step c) (3.52g) in dry methanol (60ml) was cooled to $0^{o}$ under nitrogen and treated with a solution of potassium hydroxide (0.66g) in methanol (10ml). The mixture was then allowed to warm to room temperature over 3h. The solution was treated with ether (30ml), petroleum ether (30ml) and water (44ml). The organic phase was removed and the resulting aqueous solution treated with potassium hydroxide (0.82g). After 16h at room temperature the mixture was evaporated to low volume, made up to 80ml with water and the solution washed with ethyl acetate. The aqueous phase was acidified to pH 1 with 5M hydrochloric acid and the product extracted with ethyl acetate. The organic solution was dried ($Na_2SO_4$) and evaporated to a gum (3.5g). A solution of the residue in ether yielded crystals (1.8g) of the product, mp 96-98$^{o}$.

$C_8H_{16}N_2O_3S$.

Requires C43.64, H7.27, N12.73, S14.55%

Found C43.95, H7.37, N12.73, S14.42%

Molecular weight 220.

Mass spectrum shows m/z 220 ($M^+$).

Example 16

[2-Benzyl-1-(3-mercapto-2-methyl-1-oxopropyl-2-methyl-

hydrazino]acetic acid

a) 2-Benzyl-1-formyl-2-methylhydrazine

Prepared from appropriate starting materials using the process of Example 15a). The product was isolated as an oil.

$C_9H_{12}N_2O$ Molecular weight 164.

Mass spectrum shows m/z 164 ($M^+$).

b) Ethyl [2-benzyl-1-formyl-2-methylhydrazino]acetate

Prepared from the product of step a) using the process of Example 15b). The product was isolated as an oil.

$C_{13}H_{18}N_2O_3$ Molecular weight 250.

Mass spectrum shows m/z 251 ($M^++1$).

c) Ethyl [1-(3-benzoylthio-2-methyl-1-oxopropyl)-2-benzyl-2-methylhydrazino]acetate

Prepared from the product of step b) using the method of Example 9c). The product was isolated as an oil.

$C_{23}H_{28}N_2O_4S$ Molecular weight 428.

Mass spectrum shows m/z 429 ($M^++1$).

d) [2-Benzyl-1-(3-mercapto-2-methyl-1-oxopropyl)-2-methylhydrazino]acetic acid dicyclohexylamine salt

To a stirred solution of the product of step c) (2.57g) in methanol (46ml) at $0^o$ under nitrogen was added a solution of potassium hydroxide (0.4g) in methanol (11.4ml). After 2h at room temperature ether (23ml),

petroleum ether (23ml) and water (34ml) were added. The aqueous phase was removed and treated with a solution of potassium hydroxide (600mg) in water (5ml) and the mixture stirred for 16h at room temperature. The solvents were removed by evaporation and a solution of the residue in water was washed with ethyl acetate. The aqueous phase was removed, acidified to pH 1 (2N hydrochloric acid) and the product extracted into ethyl acetate. The dried ($Na_2SO_4$) organic phase was evaporated to an oil and the product purified by chromatography on silica gel. A solution of the product in ether (15ml) was treated with dicylcohexylamine (0.52g) in ether (15ml) and the mixture evaporated to a foam. Trituration of the residue with cyclohexane (40ml) yielded the product (1.1g) as colourless crystals, mp 123-4$^{o}$.

$C_{14}H_{20}N_2O_3S$. $C_{12}H_{23}N$.

Requires C65.41, H9.01, N8.81, S6.71%

Found C65.66, H8.97, N8.52, S6.83%

Molecular weight of acid 296.

Mass spectrum shows m/z 297 ($M^{+}$+1).

Example 17

[1-(3-Mercapto-2-methyl-1-oxopropyl)-2,2-tetramethylene-hydrazino]acetic acid

a) Ethyl [1-(3-benzoylthio-2-methyl-1-oxopropyl)-2,2-tetramethylenehydrazino]acetate

Prepared from appropriate starting materials and the process of Example 9c). The product was isolated as a pale yellow oil.

$C_{19}H_{26}N_2O_4S$.

Requires C60.31, H6.88, N7.41, S8.47%

Found C60.09, H6.81, N7.12, S8.40%

Molecular weight 378.

Mass spectrum shows m/z 378 ($M^+$).

b) <u>[1-(3-Mercapto-2-methyl-1-oxopropyl)-2,2-tetramethylenehydrazino]acetic acid</u>

Prepared from the product of step a) by the process of Example 7b). The product was isolated as colourless crystals, mp 120-121$^o$.

$C_{10}H_{18}N_2O_3S$.

Requires C48.78, H7.32, N11.38, S13.00%

Found C49.13, H7.40, N11.08, S13.26%

Molecular weight of the di-trimethylsilyl derivative 390.

Mass spectrum shows m/z 390.

<u>Example 18</u>

<u>[1-(3-Mercapto-2-methyl-1-oxopropyl)-2,2-pentamethylene-hydrazino]acetic acid</u>

a) <u>Ethyl [1-(3-benzoylthio-2-methyl-1-oxopropyl)-2,2-pentamethylenehydrazino]acetate</u>

Prepared from appropriate starting materials and the process of Example 9c). The product was isolated as a

pale yellow oil.

$C_{20}H_{28}N_2O_4S$ Molecular weight 392.

Mass spectrum shows m/z 393 ($M^+$+1).

b) [1-(3-Mercapto-2-methyl-1-oxopropyl)-2,2-pentamethylene-hydrazino]acetic acid

Prepared from the product of step a) by the process of Example 7b). The product was isolated as colourless crystals, mp 112-4°.

$C_{11}H_{20}N_2O_3S$.

Requires C50.77, H7.69, N10.77, S12.31%

Found C51.15, H7.63, N10.56, S12.42%

Molecular weight 260.

Mass spectrum shows m/z 260 ($M^+$).

Example 19

(S)-[1-(3-Mercapto-2-methyl-1-oxopropyl)-2,2-pentamethylenehydrazino]acetic acid

a) Ethyl (S)-[1-(3-benzoylthio-2-methyl-1-oxopropyl)-2,2-pentamethylenehydrazino]acetate

Prepared from appropriate starting materials by the process of Example 9c). The product was isolated as a pale yellow oil.

$C_{20}H_{28}N_2O_4S$ Molecular weight 392.

Mass spectrum shows m/z 393 ($M^+$+1).

b) (S)-[1-(3-Mercapto-2-methyl-1-oxopropyl)-2,2-pentamethylenehydrazino]acetic acid

Prepared from the product of step a) by the process of Example 7b). The product was isolated as colourless crystals, mp 78-79.5°.

$C_{11}H_{20}N_2O_3S$.

Requires C50.77, H7.69, N10.77, S12.31%

Found C50.67, H7.36, N10.61, S12.11%

Molecular weight 260.

Mass spectrum shows m/z 260 ($M^+$).

Example 20

[1-(3-Mercapto-2-methyl-1-oxopropyl)-2,2-diphenylhydrazino]acetic acid

a) Benzyl [2,2-diphenylhydrazino]acetate

Prepared from appropriate starting materials by the process of Example 1a). The product was isolated as an oil.

$C_{21}H_{20}N_2O_2$ Molecular weight 332.

Mass spectrum shows m/z 332 ($M^+$).

b) Benzyl [1-(3-benzoylthio-2-methyl-1-oxopropyl)-2,2-diphenylhydrazino]acetate

Prepared from the product of step a) using the process of Example 6a). The product was isolated as an oil.

$C_{32}H_{30}N_2O_4S$.

Requires C71.38, H5.58, N5.20, S5.95%

Found C71.44, H5.64, N5.01, S6.08%

Molecular weight 538.

Mass spectrum shows m/z 538 ($M^+$).

c) [1-(3-Mercapto-2-methyl-1-oxopropyl)-2,2-diphenylhydrazino]acetic acid

Prepared from the product of step b) by the process of Example 7b). The product was isolated as colourless crystals, mp 150-4°.

$C_{18}H_{20}N_2O_3S$.

Requires C62.79, H5.81, N8.14, S9.30%

Found C62.75, H6.00, N7.83, S9.48%

Molecular weight 344.

Mass spectrum shows m/z 344 ($M^+$).

Example 21

[2-(3,4-Dichlorophenyl)-1-(3-mercapto-2-methyl-1-oxopropyl)-2-methylhydrazino]acetic acid

a) 2-(3,4-Dichlorophenyl)-1-formyl-2-methylhydrazine

Prepared from appropriate starting materials using the process of Example 9a). The product was isolated as a light-brown semi-solid.

b) Ethyl [2-(3,4-dichlorophenyl)-1-formyl-2-methylhydrazino]acetate

Prepared from the product of step a) using the process of Example 9b). The product was isolated as an orange oil.

c) Ethyl [1-(3-benzoylthio-2-methyl-1-oxopropyl)-2-

(3,4-dichlorophenyl)-2-methyl-hydrazino]acetate

Prepared from the product of step b) using the process of Example 9c). The product was isolated as colourless crystals, mp 94-95°.

$C_{22}H_{24}Cl_2N_2O_4S$.

Requires C54.66, H4.97, N5.80%

Found C54.53, H4.93, N5.73%

d) [2-(3,4-Dichlorophenyl)-1-(3-mercapto-2-methyl-1-oxopropyl)-2-methylhydrazino]acetic acid

Prepared from the product of step c) using the process of Example 7b). The product was isolated as colourless crystals, mp 135-6°.

$C_{13}H_{16}Cl_2N_2O_3S$.

Requires C44.45, H4.59, N7.98%

Found C44.63, H4.62, N7.78%

Other Compounds of formula I which may be mentioned are:-

(S)-[1-[N-(1-Ethoxycarbonyl-2-phenylpropyl)]glycyl-2-methyl-2-phenylhydrazino]acetic acid.

(S)-[1-[N-(1-Ethoxycarbonyl-2-phenylpropyl)]alanyl-2-methyl-2-phenylhydrazino]acetic acid.

(S)-[1-[N-(1-Ethoxycarbonyl-2-phenylpropyl)]glycyl-2,2-diethylhydrazino]acetic acid.

(S)-[1-[N-(1-Ethoxycarbonyl-2-phenylpropyl)]alanyl-2,2-diethylhydrazino]acetic acid.

and the 2,2-dimethyl anologous of the last two compounds.

(S)-[1-[N-(1-Ethoxycarbonyl-2-phenylpropyl)]alanyl-2-benzyl-2-methylhydrazino]acetic acid.

[1-(3-Benzoylthio-2-methyl-1-oxopropyl)-2,2-dimethylhydrazino]acetic acid.

Example A

In vitro Assay of inhibitors of Angiotensin Converting Enzyme

The method is based upon that of Cushman and Cheung (1971) but uses a radioactive substrate [glycine-1-$^{14}$C] hippuryl-L-histidyl-L-leucine (HHL) whose hydrolysis may be determined by liquid scintillation counting of released [$^{14}$C]-hippuric acid. Hydrolysis of 2mM HHL by an extract of rabbit lung acetone powder (Sigma) over a 30 min incubation period at 37$^{o}$ is followed by acidification of the reaction mixture and extraction of [$^{14}$C]hippurate with ethyl acetate.

Potential inhibitors are tested initially at 0.01mM and if found active are retested at lower concentrations to determine an $IC_{50}$. Dimethyl sulphoxide at 1% final concentration may be used as a solubility aid without affecting enzyme activity. Compounds of special interest are studied at a range of substrate and inhibitor concentrations to determine the type of inhibition and are also tested against other enzymes, e.g. carboxypeptidase A

to establish their specificity for ACE.

Example B

Antihypertensive effects were investigated in conscious spontaneously hypertensive rats (SHR) of the Okamoto strain. Systolic blood pressure and heart rate were measured by the tail cuff method using an electrosphygmomanometer 1 hour before and 1, 3, 5 and 24 hours after oral dosing with the compound (dose range 0.1 - 100 mg/kg p.o.). Percentage changes in each parameter were measured with respect to pretreatment control values.

7788H(ir)/jaa/jed

What we claim is:-

1. A compound of formula I,

$$ZCHRCON(R_4NR_5)CHR_6COOH \qquad I$$

in which Z is $R_2CH(COOH)NH-$ or $R_1SCH_2-$,

$R_1$ is hydrogen or $R_8CO-$,

$R_8$ is alkyl C1 to 10 or phenyl,

R is hydrogen, alkyl C1 to 6, or alkyl C1 to 6 substituted by phenyl, $-NH_2$, -COOH or by alkyl C1 to 6-thio,

$R_2$ is hydrogen, alkyl C1 to 10, cycloalkyl C3 to 10, phenyl, phenylalkyl C7 to 12, alkoxy C1 to 6 - alkyl C1 to 6, alkyl C1 to 6 - thioalkyl C1 to 6, phenyloxyalkyl C7 to 12 or phenylthioalkyl C7 to 12,

$R_4$ and $R_5$, which may be the same or different, are each hydrogen, alkyl C1 to 10, cycloalkyl C3 to 10, phenylalkyl C7 to 12, or phenyl; the phenyl and phenylalkyl each optionally being substituted by one or more alkyl C1 to 6, alkoxy C1 to 6 or halogen groups,

or $R_4$ and $R_5$ together form a $-(CH_2)_4-$ or $-(CH_2)_5-$ chain, and

$R_6$ is hydrogen, alkyl C1 to 6 or phenylalkyl C7 to 12,

and pharmaceutically acceptable salts, esters and amides

thereof.

2. A compound according to Claim 1, wherein Z is $R_1SCH_2$-.

3. A compound according to Claim 1, wherein when Z is $R_2CH(COOH)NH$- the partial structure -NHCHRCO- in formula I is a part of a naturally occurring amino acid.

4. A compound according to any one of the preceding claims, wherein when any of $R_2$, $R_4$, $R_5$ or $R_8$ represent alkyl they individually contain up to and including 6 carbon atoms.

5. A compound according to any one of the preceding claims, wherein R is hydrogen or methyl.

6. A compound according to any one of Claims 1 or 3 to 6, wherein $R_2$ is phenylalkyl C7 to 10 or alkyl C1 to 4-thioalkyl C1 to 4.

7. A compound according to Claim 6, wherein $R_2$ is benzyl, phenylethyl or 2-methylthioethyl.

8. A compound according to any one of the preceding claims, wherein $R_4$ and $R_5$ are alkyl C1 to 6, phenylalkyl C7 to 10, halophenyl, alkyl C1 to 6-phenyl or together form a $-(CH_2)_4-$ or $-(CH_2)_5-$ chain.

9. A compound according to Claim 8, wherein $R_4$ and $R_5$ are methyl, ethyl, benzyl, phenyl, 4-chlorophenyl, 3,4-dichlorophenyl, 4-methylphenyl or together form a $-(CH_2)_4-$ or $-(CH_2)_5-$ chain.

10. A compound according to any one of the preceding claims, wherein $R_6$ is hydrogen, methyl or phenylethyl.

11. A compound according to any one of Claims 1, 2 or 4 to 10 inclusive, wherein $R_1$ is hydrogen, benzoyl or alkanoyl C2 to 6.

12. A compound according to Claim 11, wherein $R_1$ is hydrogen, benzoyl, acetyl or pivaloyl.

13. A compound according to any one of the preceding claims, in the form of an alkyl C1 to 6 ester or an ester with benzyl alcohol.

14. A compound according to any one of Claims 1, 3 to 10 or 13, wherein Z is $R_2CH(COOH)NH-$ and the -COOH group adjacent to the $R_2$ group is esterified.

15. A compound of formula Ia,

$$R_2CH(COOH)NHCHRCON(NR_4R_5)CHR_6COOH \qquad Ia$$

in which R is hydrogen or alkyl C1 to 6,

$R_2$ is hydrogen, alkyl C1 to 10 or phenylalkyl C7 to 12,

$R_4$ and $R_5$, which may be the same or different, are each hydrogen, alkyl C1 to 10, cycloalkyl C3 to C10, phenylalkyl C7 to 12, or phenyl, the phenyl and phenylalkyl each optionally being substituted by one or more alkyl C1 to 6, alkoxy C1 to 6 or halogen groups,

or $R_4$ and $R_5$ together form a $-(CH_2)_4-$ or $-(CH_2)_5-$ chain, and

$R_6$ is hydrogen or alkyl C1 to 6,

and pharmaceutically acceptable salts and esters thereof.

16. A compound of formula Ib,

$$R_1SCH_2CHR\text{-}CON(NR_4R_5)CHR_6COOH \qquad \text{Ib}$$

in which $R_1$ is hydrogen or $R_8CO-$,

$R_8$ is alkyl C1 to 10, or phenyl,

R is hydrogen or alkyl C1 to 6,

$R_4$ and $R_5$, which may be the same or different, are each, hydrogen, alkyl C1 to 10, cycloalkyl C3 to 10, phenylalkyl C7 to 12, or phenyl; the phenyl and phenylalkyl each optionally being substituted by one or more alkyl C1 to 6, alkoxy C1 to 6 or halogen groups,

or $R_4$ and $R_5$ may together form a $-(CH_2)_4-$ or $-(CH_2)_5-$ chain, and

$R_6$ is hydrogen, or alkyl C1 to 10,

and pharmaceutically acceptable salts and esters thereof.

17. A compound of formula Ic,

$$R_1SCH_2CHR\text{-}CON(NR_4R_5)CHR_6COOH \qquad \text{Ic}$$

in which $R_1$ is hydrogen or $R_8CO-$

$R_8$ is alkyl C1 to 10, or phenyl,

R is hydrogen or alkyl C1 to 10,

$R_4$ and $R_5$, which may be the same or different, are each, alkyl C1 to 10, phenylalkyl C7 to 12 or phenyl; the phenyl optionally being substituted by one or more alkyl C1 to 6, alkoxy C1 to 6 or halogen groups, and

$R_6$ is hydrogen, or alkyl C1 to 10,

and pharmaceutically acceptable salts, esters and amides thereof.

18. A compound of formula Id,

$R_2CH(COOH)NHCHRCON(NR_4R_5)CH_2COOH$ Id

in which R is hydrogen, alkyl C1 to 6, or alkyl C1 to 6 substituted by phenyl, $-NH_2$, -COOH or by alkyl C1 to 6- thio,

$R_2$ is hydrogen, alkyl C1 to 10, cycloalkyl C3 to 10, phenyl, phenylalkyl C7 to 12, alkoxy C1 to 6 - alkyl C1 to 6, alkyl C1 to 6 - thioalkyl C1 to 6, phenyloxyalkyl C7 to 12 or phenylthioalkyl C7 to 12,

$R_4$ and $R_5$, which may be the same or different, are each hydrogen, alkyl C1 to 6, phenylalkyl C7 to 12, or phenyl, the phenyl optionally being substituted by one or more alkyl C1 to 6, alkoxy C1 to 6 or halogen groups, or $R_4$ and $R_5$ together form a $-(CH_2)_4-$ chain,

and pharmaceutically acceptable salts, esters and

amides thereof.

19. A compound according to Claim 1 wherein $R_2$ is cycloalkyl C3 to 10 or phenylthioalkyl C7 to 12.

20. (S)[1-[N-(1-Carboxy-2-phenylethyl)glycyl]-2-methyl-2-phenylhydrazino]acetic acid

Benzyl (S)-[1-N-(1-benzyloxycarbonyl-2-phenylethyl)-glycyl]-2-methyl-2-phenylhydrazino]acetate

Benzyl (S)[1-[N-[1-methoxycarbonyl-3-(methylthio)propyl]glycyl]-2-methyl-2-phenylhydrazino]acetate

(S)-[1-[N-[1-Carboxy-3-(methylthio)propyl]-glycyl]-2-methyl-2-phenylhydrazino]acetic acid

Benzyl[1-[N-(1-benzyloxycarbonyl-3-phenylpropyl)-glycyl]-2-methyl-2-phenylhydrazino]acetate

[1-(N-(1-Carboxy-3-phenylpropyl)glycyl)-2-methyl-2-phenylhydrazino]acetic acid

Benzyl [1-(N-(1-benzyloxycarbonyl-2-phenylethyl)-alanyl)-2-methyl-2-phenylhydrazino]acetate

Benzyl [1-(N-(1-benzyloxycarbonyl-3-phenylpropyl)alanyl)-2-methyl-2-phenyl-hydrazino]acetate

Benzyl [1-(3-benzoylthio-1-oxopropyl)-2-methyl-2-phenylhydrazino]acetate

[1-(3-Mercapto-1-oxopropyl)-2-methyl-2-phenylhydrazino]acetic acid

Benzyl [1-(3-benzoylthio-2-methyl-1-oxopropyl)-2-methyl-2-phenylhydrazino]acetate

[1-(3-Mercapto-2-methyl-1-oxopropyl)-2-methyl-2-phenylhydrazino]acetic acid

tert-Butyl [1-(3-benzoylthio-2-methyl-1-oxopropyl)-2-methyl-2-phenylhydrazino]acetate

[1-(3-Benzoylthio-2-methyl-1-oxopropyl)-2-methyl-2-phenylhydrazino]acetic acid

Ethyl [1-(3-benzoylthio-2-methyl-1-oxopropyl)-2-(4-chlorophenyl)-2-methylhydrazino]acetate

[2-(4-Chlorophenyl)-1-(3-mercapto-2-methyl-1-oxopropyl)-2-methylhydrazino]acetic acid

tert-Butyl (S)-[1-(3-acetylthio-2-methyl-1-oxopropyl)-2-methyl-2-phenylhydrazino]acetate

(S)-[1-(3-Acetylthio-2-methyl-1-oxopropyl)-2-methyl-2-phenylhydrazino]acetic acid

Benzyl (S)-[1-(3-benzoylthio-2-methyl-1-oxopropyl)-2-methyl-2-phenylhydrazino]acetate

(S)-[1-(3-Mercapto-2-methyl-1-oxopropyl)-2-methyl-2-phenylhydrazino]acetic acid

Ethyl [1-(3-benzoylthio-2-methyl-1-oxopropyl)-2-methyl-2-(4-methylphenyl)-hydrazino]acetate

[1-(3-Mercapto-2-methyl-1-oxopropyl)-2-methyl-2-(4-methylphenyl)hydrazino]acetic acid

Ethyl 2-[1-(3-benzoylthio-1-oxopropyl)-2-methyl-2-phenylhydrazino]propanoate

2-[1-(3-Mercapto-1-oxopropyl)-2-methyl-2-

phenylhydrazino]propanoic acid

Ethyl 2-[1-(3-benzoylthio-1-oxopropyl)-2-methyl-2-phenylhydrazino]-4-phenylbutyrate

2-[1-(3-Mercapto-1-oxopropyl)-2-methyl-2-phenylhydrazino]-4-phenylbutyric acid

Ethyl [1-(3-benzoylthio-2-methyl-1-oxopropyl)-2,2-dimethylhydrazino]acetate

[1-(3-Mercapto-2-methyl-1-oxopropyl)-2,2-dimethylhydrazino]acetic acid

Ethyl [1-(3-benzoylthio-2-methyl-1-oxopropyl)-2-benzyl-2-methylhydrazino]acetate

[2-Benzyl-1-(3-mercapto-2-methyl-1-oxopropyl)-2-methylhydrazino]acetic acid

Ethyl [1-(3-benzoylthio-2-methyl-1-oxopropyl)-2,2-tetramethylenehydrazino]acetate

[1-(3-Mercapto-2-methyl-1-oxopropyl)-2,2-tetramethylenehydrazino]acetic acid

Ethyl [1-(3-benzoylthio-2-methyl-1-oxopropyl)-2,2-pentamethylenehydrazino]acetate

[1-(3-Mercapto-2-methyl-1-oxopropyl)-2,2-pentamethylene-hydrazino]acetic acid

Ethyl (S)-[1-(3-benzoylthio-2-methyl-1-oxopropyl)-2,2-pentamethylenehydrazino]acetate

(S)-[1-(3-Mercapto-2-methyl-1-oxopropyl-2,2-pentamethylenehydrazino]acetic acid

Benzyl [1-(3-benzoylthio-2-methyl-1-oxopropyl)-2,2-diphenylhydrazino]acetate

[1-(3-Mercapto-2-methyl-1-oxopropyl)-2,2-diphenylhydrazino]acetic acid

Ethyl [1-(3-benzoylthio-2-methyl-1-oxopropyl)-2-(3,4-dichlorophenyl)-2-methyl-hydrazino]acetate

[2-(3,4-Dichlorophenyl)-1-(3-mercapto-2-methyl-1-oxopropyl)-2-methylhydrazino]acetic acid

(S)-[1-[N-(1-Ethoxycarbonyl-2-phenylpropyl)]glycyl-2-methyl-2-phenylhydrazino]acetic acid

(S)-[1-[N-(1-Ethoxycarbonyl-2-phenylpropyl)]alanyl-2-methyl-2-phenylhydrazino]acetic acid

(S)-[1-[N-(1-Ethoxycarbonyl-2-phenylpropyl)]glycyl-2,2-diethylhydrazino]acetic acid

(S)-[1-[N-(1-Ethoxycarbonyl-2-phenylpropyl)]alanyl-2,2-diethylhydrazino]acetic acid

(S)-[1-[N-(1-Ethoxycarbonyl-2-phenylpropyl)]glycyl-2,2-dimethylhydrazino]acetic acid

(S)-[1-[N-(1-Ethoxycarbonyl-2-phenylpropyl)]analyl-2,2-dimethylhydrazino]acetic acid

(S)-[1-[N-(1-Ethoxycarbonyl-2-phenylpropyl)]alanyl-2-benzyl-2-methylhydrazino]acetic acid

[1-(3-Benzoylthio-2-methyl-1-oxopropyl)-2,2-dimethylhydrazino]acetic acid

or a pharmaceutically acceptable salt of any one of

those compounds having a free acidic or basic group.

21. A compound according to any one of the preceding claims, wherein any asymmetric carbon atoms are in the S configuration.

22. A process for the production of a compound of formula I, as defined in Claim 1, or a pharmaceutically acceptable salt, ester or amide thereof, which comprises

a) removal of a protecting group from a compound of formula I in which one or more of the amino or carboxylic acid groups is protected,

b) production of a compound of formula I in which Z is $R_2CH(COOH)NH-$, or a salt or ester thereof, by reaction of a compound of formula II,

$$R_2CH(NH_2)COOR_3 \qquad II$$

in which $R_2$ is as defined above and $R_3$ is hydrogen or a protecting group,

with a compound of formula III,

$$XCHRCON(R_4NR_5)CHR_6COOH \qquad III$$

or a salt, ester or protected derivative thereof, in which R, $R_4$, $R_5$ and $R_6$ are as defined above,

and

X is a good leaving group,

c) production of a compound of formula I in which $R_1$ is $R_8CO-$ by reaction of a compound of formula IV,

$R_8CO-S-CH_2CHRCOX$ IV

in which X, R and $R_8$ are defined above, with a compound of formula V,

$HN(NR_4R_5)CHR_6COOH$ V

or a salt, ester or protected derivative thereof, in which $R_4$,$R_5$ and $R_6$ are as defined above, or

d) production of a compound of formula I in which $R_1$ is hydrogen by selective cleavage of a corresponding compound of formula I in which $R_1$ is $R_8CO-$,

and where desired or necessary deprotecting the resulting compound, or converting a compound of formula I to a pharmaceutically acceptable salt, ester or amide thereof or vice versa.

23. A pharmaceutical composition comprising a compound according to any one of Claims 1 to 20 in admixture with a pharmaceutically acceptable adjuvant, diluent or carrier.

24. Use of a compound according to any one of Claims 1 to 20 as a pharmaceutical.

What we claim is:-

1. A process for the production of a compound of formula I,

$$ZCHRCON(R_4NR_5)CHR_6COOH \qquad I$$

in which Z is $R_2CH(COOH)NH-$ or $R_1SCH_2-$,

$R_1$ is hydrogen or $R_8CO-$,

$R_8$ is alkyl C1 to 10 or phenyl,

R is hydrogen, alkyl C1 to 6, or alkyl C1 to 6 substituted by phenyl, $-NH_2$, -COOH or by alkyl C1 to 6-thio,

$R_2$ is hydrogen, alkyl C1 to 10, cycloalkyl C3 to 10, phenyl, phenylalkyl C7 to 12, alkoxy C1 to 6 - alkyl C1 to 6, alkyl C1 to 6 - thioalkyl C1 to 6, phenyloxyalkyl C7 to 12 or phenylthioalkyl C7 to 12,

$R_4$ and $R_5$, which may be the same or different, are each hydrogen, alkyl C1 to 10, cycloalkyl C3 to 10, phenylalkyl C7 to 12, or phenyl; the phenyl and phenylalkyl each optionally being substituted by one or more alkyl C1 to 6, alkoxy C1 to 6 or halogen groups,

or $R_4$ and $R_5$ together form a $-(CH_2)_4-$ or $-(CH_2)_5-$ chain, and

$R_6$ is hydrogen, alkyl C1 to 6 or phenylalkyl C7 to 12,

or a pharmaceutically acceptable salt, ester or amide

thereof, which comprises

a) removal of a protecting group from a compound of formula I in which one or more of the amino or carboxylic acid groups is protected,

b) production of a compound of formula I in which Z is $R_2CH(COOH)NH-$, or a salt or ester thereof, by reaction of a compound of formula II,

$$R_2CH(NH_2)COOR_3 \qquad II$$

in which $R_2$ is as defined above and $R_3$ is hydrogen or a protecting group,

with a compound of formula III,

$$XCHRCON(R_4NR_5)CHR_6COOH \qquad III$$

or a salt, ester or protected derivative thereof,

in which R, $R_4$, $R_5$ and $R_6$ are as defined above, and

X is a good leaving group,

c) production of a compound of formula I in which $R_1$ is $R_8CO-$ by reaction of a compound of formula IV,

$$R_8CO-S-CH_2CHRCOX \qquad IV$$

in which X, R and $R_8$ are defined above, with a compound of formula V,

$HN(NR_4R_5)CHR_6COOH$ V

or a salt, ester or protected derivative thereof, in which $R_4$,$R_5$ and $R_6$ are as defined above, or

d) production of a compound of formula I in which $R_1$ is hydrogen by selective cleavage of a corresponding compound of formula I in which $R_1$ is $R_8CO-$,

and where desired or necessary deprotecting the resulting compound, or converting a compound of formula I to a pharmaceutically acceptable salt, ester or amide thereof or vice versa.

2. A process according to Claim 1, wherein Z is $R_1SCH_2-$.

3. A process according to Claim 1, wherein when Z is $R_2CH(COOH)NH-$ the partial structure -NHCHRCO- in formula I is a part of a naturally occurring amino acid.

4. A process according to any one of the preceding claims, wherein when any of $R_2$, $R_4$, $R_5$ or $R_8$ represent alkyl they individually contain up to and including 6 carbon atoms.

5. A process according to any one of the preceding claims, wherein R is hydrogen or methyl.

6. A process according to any one of Claims 1 or 3 to 6, wherein $R_2$ is phenylalkyl C7 to 10 or alkyl C1 to 4-thioalkyl C1 to 4.

7. A process according to Claim 6, wherein $R_2$ is benzyl, phenylethyl or 2-methylthioethyl.

8. A process according to any one of the preceding claims, wherein $R_4$ and $R_5$ are alkyl C1 to 6, phenylalkyl C7 to 10, halophenyl, alkyl C1 to 6-phenyl or together form a $-(CH_2)_4-$ or $-(CH_2)_5-$ chain.

9. A process according to Claim 8, wherein $R_4$ and $R_5$ are methyl, ethyl, benzyl, phenyl, 4-chlorophenyl, 3,4-dichlorophenyl, 4-methylphenyl or together form a $-(CH_2)_4-$ or $-(CH_2)_5-$ chain.

10. A process according to any one of the preceding claims, wherein $R_6$ is hydrogen, methyl or phenylethyl.

11. A process according to any one of Claims 1, 2 or 4 to 10 inclusive, wherein $R_1$ is hydrogen, benzoyl or alkanoyl C2 to 6.

12. A process according to Claim 11, wherein $R_1$ is hydrogen, benzoyl, acetyl or pivaloyl.

13. A process according to any one of the preceding claims, wherein the compound of formula I is in the form of an alkyl C1 to 6 ester or an ester with benzyl alcohol.

14. A process according to any one of Claims 1, 3 to 10 or 13, wherein Z is $R_2CH(COOH)NH-$ and the -COOH group

adjacent to the $R_2$ group is esterified.

15. A process according to Claim 1 wherein the compound of formula I is of formula Ic,

$R_1SCH_2CHR-CON(NR_4R_5)CHR_6COOH$ Ic

in which $R_1$ is hydrogen or $R_8CO-$

$R_8$ is alkyl C1 to 10, or phenyl,

R is hydrogen or alkyl C1 to 10,

$R_4$ and $R_5$, which may be the same or different, are each, alkyl C1 to 10, phenylalkyl C7 to 12 or phenyl; the phenyl optionally being substituted by one or more alkyl C1 to 6, alkoxy C1 to 6 or halogen groups, and

$R_6$ is hydrogen, or alkyl C1 to 10,

or a pharmaceutically acceptable salt, ester or amide thereof.

16. A process according to Claim 1, wherein the compound of formula I is of formula Id,

$R_2CH(COOH)NHCHRCON(NR_4R_5)CH_2COOH$ Id

in which R is hydrogen, alkyl C1 to 6, or alkyl C1 to 6 substituted by phenyl, $-NH_2$, -COOH or by alkyl C1 to 6- thio,

$R_2$ is hydrogen, alkyl C1 to 10, cycloalkyl C3 to 10, phenyl, phenylalkyl C7 to 12, alkoxy C1 to 6 - alkyl C1 to 6, alkyl C1 to 6 - thioalkyl C1 to 6, phenyloxyalkyl C7 to 12 or phenylthioalkyl C7 to 12,

$R_4$ and $R_5$, which may be the same or different, are each hydrogen, alkyl C1 to 6, phenylalkyl C7 to 12, or phenyl, the phenyl optionally being substituted by one or more alkyl C1 to 6, alkoxy C1 to 6 or halogen groups, or $R_4$ and $R_5$ together form a $-(CH_2)_4-$ chain,

or a pharmaceutically acceptable salt, ester or amide thereof.

17. A process according to Claim 1 wherein $R_2$ is cycloalkyl C3 to 10 or phenylthioalkyl C7 to 12.

7906H/jed